# EUROPEAN PATENT SPECIFICATION

(11) **EP 2 855 497 B1**
(45) Date of publication and mention of the grant of the patent: **20.04.2016**
(21) Application number: 13725159.1
(22) Date of filing: 27.05.2013
(51) Int. Cl.: C07H 1/00, C07H 19/06, A61P 31/12

(54) **PROCESS FOR THE PREPARATION OF 2-DEOXY-2-FLUORO-2-METHYL-D-RIBOFURANOSYL NUCLEOSIDE COMPOUNDS**
VERFAHREN ZUR HERSTELLUNG VON 2-DEOXY-2-FLUOR-2-METHYL-D-RIBOFURANOSYLNUKLEOSIDVERBINDUNGEN
PROCÉDÉ POUR LA PRÉPARATION DE COMPOSÉS DE NUCLÉOSIDE DE 2-DÉOXY-2-FLUORO-2-MÉTHYL-D-RIBOFURANOSYLE

(30) Priority: 29.05.2012 EP 12169760
(43) Date of publication of application: 08.04.2015
(73) Proprietor: F.Hoffmann-La Roche AG, 4070 Basel (CH)
(72) Inventor: CARR, Robert, Effingham, South Carolina 29541 (US); HILDBRAND, Stefan, CH-4460 Gelterkinden (CH); HODGES, Mark L., Florence, South Carolina 29501 (US); KAMMERER, Michael, CH-4051 Basel (CH); LANG, John F., Florence, South Carolina 29505 (US); LAWRIMORE III, William J., Florence, South Carolina 29505 (US); NGUYEN, Dieu, Wilmington, Massachusetts 01887 (US)
(74) Representative: Rauber, Beat
(86) International application number: PCT/EP2013/060836
(87) International publication number: WO 2013/178571

(56) References cited:
- EP-A1- 2 048 151
- WO-A1-2008/045419
- WO-A2-2006/031725

## Description

The present invention relates to an improved process for the preparation of (2'R)-2'-deoxy-2'-fluoro-2'-methylcytidine derivatives of formula I 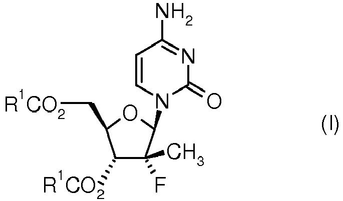 wherein R¹ is selected from C₁₋₄-alkyl,
which have the potential to be useful as prodrugs for potent inhibitors of the Hepatitis C Virus (HCV) NS5B polymerase (PCT Int. Publ. WO 2007/065829).

The preparation of the (2'R)-2'-deoxy-2'-fluoro-2'-methylcytidine derivatives of formula I can follow the known steps:
a) transforming the (2R)-2-deoxy-2-fluoro-2-methyl-D-ribonolactone derivative of formula II 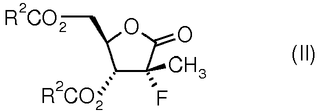 wherein R² is phenyl or C₁₋₄-alkyl
   into the (2R)-2-deoxy-2-fluoro-2-methyl-D-ribofuranosyl chloride of formula III 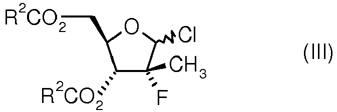 wherein R² is phenyl or C₁₋₄-alkyl
b) coupling the (2R)-2-deoxy-2-fluoro-2-methyl-D-ribofuranosyl chloride of formula III with N-benzoyl cytosine of formula VIa 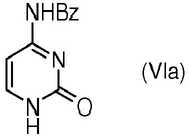 to form the (2'R)-N-benzoyl-2'-deoxy-2'-fluoro-2'-methyl-cytidine derivative of formula IV 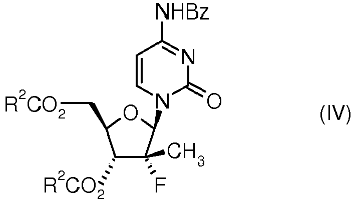 wherein R² is as above and Bz is benzoyl
c) alcoholysis of (2'R)-N-benzoyl-2'-deoxy-2'-fluoro-2'-methyl-cytidine derivative of formula IV to afford the (2'R)-2'-deoxy-2'-fluoro-2'-methyl-cytidine of formula V 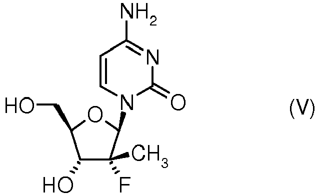 and finally
d) acylating the (2'R)-2'-deoxy-2'-fluoro-2'-methyl-cytidine of formula V to form the (2'R)-2'-deoxy-2'-fluoro-2'-methylcytidine derivative of formula I.

It was found that the crucial step for a technical scale synthesis of the (2'R)-2'-deoxy-2'-fluoro-2'-methylcytidine derivatives of formula I is the coupling step b). According to PCT Int. Appl. WO 2008/045419 this step requires the use of substantial amounts of chlorobenzene as solvent. Due to the corrosive nature of this solvent reservation exists to apply the solvent on large scale processes. It was further observed that the quench was difficult to control regarding exothermy and HCl release. In addition the subsequent filtration of the precipitated excess of N-benzoyl cytosine proved to be very slow and accordingly resulted in a limiting factor regarding scale up capacity. Other examples of pyrimidine nucleoside synthesis are disclosed in EP2048151A and WO2006/031725.

Object of the present invention therefore was to improve synthesis step b) in such a manner that the process can be applied on technical scale.

The object of the invention could be achieved with the process of the present invention which comprises the preparation of the (2'R)-2'-deoxy-2'-fluoro-2'-methylcytidine derivative of formula I 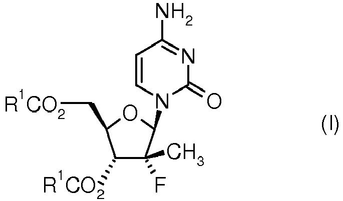 wherein R¹ is selected from C₁₋₄-alkyl
comprising the steps
a) transforming the (2R)-2-deoxy-2-fluoro-2-methyl-D-ribonolactone derivative of formula II 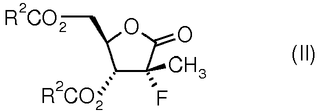 wherein R² is phenyl or C₁₋₄-alkyl
   into the (2R)-2-deoxy-2-fluoro-2-methyl-D-ribofuranosyl chloride of formula III 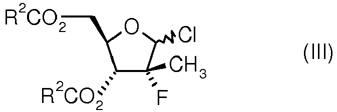 wherein R² is phenyl or C₁₋₄-alkyl
b) coupling the (2R)-2-deoxy-2-fluoro-2-methyl-D-ribofuranosyl chloride of formula III with N-benzoyl cytosine of formula VIa 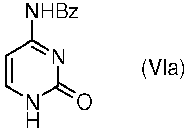 to form the (2'R)-N-benzoyl-2'-deoxy-2'-fluoro-2'-methyl-cytidine derivative of formula IV 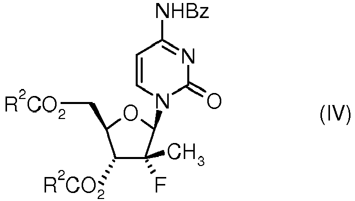 wherein R² is as above and Bz is benzoyl
c) alcoholysis of (2'R)-N-benzoyl-2'-deoxy-2'-fluoro-2'-methyl-cytidine derivative of formula IV to afford the (2'R)-2'-deoxy-2'-fluoro-2'-methyl-cytidine of formula V 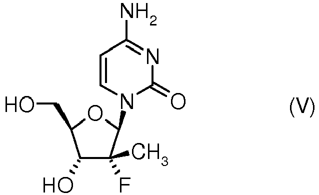 and
d) acylating the (2'R)-2'-deoxy-2'-fluoro-2'-methyl-cytidine of formula V to form the (2'R)-2'-deoxy-2'-fluoro-2'-methylcytidine derivative of formula I,
characterized in that the coupling step b) comprises
b₁) the silylation of N-benzoyl cytosine of formula VIa 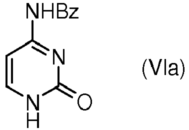 to form the silylated N-benzoyl cytosine of formula VIb 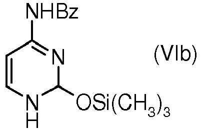 in the presence of a C₃₋₄-alkylacetate as solvent and
b₂) the coupling of the silylated N-benzoyl cytosine of formula VIb with the (2R)-2-deoxy-2-fluoro-2-methyl-D-ribofuranosyl chloride of formula III 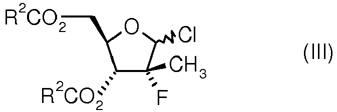 wherein R² is phenyl or C₁₋₄-alkyl,
   to form the (2'R)-N-benzoyl-2'-deoxy-2'-fluoro-2'-methyl-cytidine derivative of formula IV 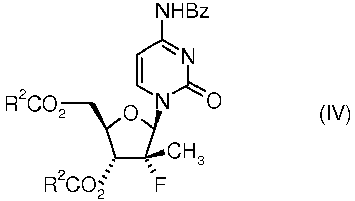 wherein R² is as above and Bz is benzoyl,
   in the presence of dichloromethane as solvent and a Lewis acid.

The following definitions are set forth to illustrate and define the meaning and scope of the various terms used to describe the invention herein.

The term "C₁₋₄-alkyl" as used herein denotes an unbranched or branched chain, saturated, monovalent hydrocarbon residue containing 1 to 4 carbon atoms, particularly methyl, ethyl, n-propyl, i-propyl, n-butyl, sec-butyl or t-butyl.

The term "C₃₋₄-alkyl" as used herein denotes an unbranched or branched chain, saturated, monovalent hydrocarbon residue containing 3 to 4 carbon atoms, particularly n-propyl, i-propyl, n-butyl, sec-butyl or t-butyl, more particularly i-propyl or n-butyl.

### Step a)

The transformation in step a) comprises a reduction in the presence of a reducing agent and a subsequent chlorination in the presence of chlorinating agent.

The reducing agent bis-(2-methoxyethoxy) (2,2,2,-trifluoro ethoxy) aluminum hydride is as a rule preformed from sodium bis-(2-methoxyethoxy) aluminum hydride, which is commercially available under the trade name Red-Al (Vitride®, solution in toluene) and trifluoroethanol.

The reduction usually takes place in an organic solvent such as in toluene at a reaction temperature of 0°C to -30°C.

After completion of the reduction the reaction mixture is subjected to the chlorination reaction.

The chlorinating agent is as a rule selected from sulfuryl chloride, thionyl chloride or phosphorus oxychloride.

Preferably sulfuryl chloride in the presence of catalytic amounts of tetrabutyl ammonium bromide is used.

The addition of the chlorinating agent as a rule takes place at a temperature of - 20°C to 0°C, thereafter the reaction can continue expediently at a reaction temperature between 20°C and 30°C.

The (2R)-2-deoxy-2-fluoro-2-methyl-D-ribofuranosyl chloride of formula III can be separated from the reaction mixture applying techniques known to the skilled in the art.

### Step b)

Coupling step b) is characterized by the steps
b₁) the silylation of N-benzoyl cytosine of formula VIa 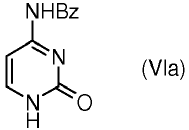 to form the silylated N-benzoyl cytosine of formula VIb 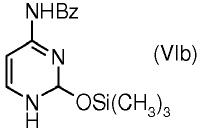 in the presence of a C₃₋₄-alkylacetate as solvent and
b₂) the coupling of the silylated N-benzoyl cytosine of formula VIb with the (2R)-2-deoxy-2-fluoro-2-methyl-D-ribofuranosyl chloride of formula III 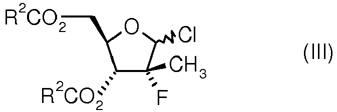 wherein R² is phenyl or C₁₋₄-alkyl,
   to form the (2'R)-N-benzoyl-2'-deoxy-2'-fluoro-2'-methyl-cytidine derivative of formula IV 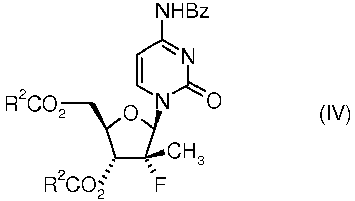 wherein R² is as above and Bz is benzoyl,
   in the presence of dichloromethane as solvent and a Lewis acid.

### Step b₁

The silylation can be performed with a suitable silylating agent such as with hexamethyldisilazane usually in the presence of ammonium sulfate.

Suitable C₃₋₄-alkylacetate solvents are i-propyl or n-butyl acetate.

The reaction as a rule takes place at temperatures of higher than 85°C, i.e. particularly at the reflux temperature of the solvent, for about 3 h to 8 h.

The resulting solution of the silylated N-benzoyl cytosine of formula VIb can, suitably after concentration, be directly used for the subsequent reaction step b₂).

### Step b₂

For step b₂) the former solvent is completely exchanged with dichloromethane.

Common Lewis acids known in the art are suitable for the conversion in step b₂). Particular good results have been achieved with tin tetrachloride.

The reaction is usually performed at a reaction temperature of 70°C to 90°C and a pressure of 2 bar to 3 bar, more particularly at a reaction temperature of 75°C to 85°C and at a pressure of 2.5 bar.

In a further particular embodiment the reaction mixture, after completion of the coupling reaction in step b₂), is quenched by adding it to a mixture of acetic acid and water of 97 : 3 (w/w) to 80 : 20 (w/w), more particularly of 95 : 5 (w/w) to 90 : 10 (w/w), at a temperature of 10°C to 30°C, more particularly at a temperature of 15°C to 25°C.

In a further particular embodiment the (2'R)-N-benzoyl-2'-deoxy-2'-fluoro-2'-methyl-cytidine derivative of formula IV, so obtained in step b₂) can be further purified by multiple extractions of the tin with a mixture of water and acetic acid and subsequent crystallization by replacing partly of the dichloromethane by methanol.

The ratio of water and acetic acid in the mixture expediently is 1 to 3 : 1 (v/v).

The extractions are repeated until the tin content in the isolated product is reproducibly <20 ppm. As a rule this target can be reached with 3 to 4 extraction cycles.

The ratio of methanol and dichloromethane in the mixture for the crystallization is usually 2 to 5 : 1 (w/w).

### Step c)

The alcoholysis in step c) is performed in the presence of a base and an alcohol as solvent.

Suitable bases are organic bases like alkali metal alkoxides, particularly sodium methoxide.

In a particular embodiment 0.03 eq. to 0.10 eq. sodium methoxide in methanol as solvent is used.

The alcoholysis reaction is usually performed at a reaction temperature of 50°C to 65°C.

Upon completion of the alcoholysis the (*2'R*)-2'-deoxy-2'-fluoro-2'-methyl-cytidine of formula V can as a rule be separated from the reaction mixture by applying techniques known to the skilled in the art, for instance by crystallization from isopropanol/methanol.

### Step d)

The acylation in step d) is as a rule performed with a C₁₋₄-alkanoyl chloride in the presence of an organic solvent/water mixture at temperatures of -5°C and 5°C.

In a particular embodiment isobutyryl chloride is the selected C₁₋₄-alkanoyl chloride and tetrahydrofuran is the selected organic solvent.

The isolation of the (2'R)-2'-deoxy-2'-fluoro-2'-methylcytidine derivative of formula I from the reaction mixture can follow methods known to the skilled in the art, for instance by an extraction of the neutralized reaction mixture with ethyl acetate and subsequent crystallization in a mixture of a C₁₋₄-alcohol and n-heptane. Suitable C₁₋₄-alcohols are methanol, ethanol and i-propanol. In a particular embodiment the crystallization is performed with a mixture of i-propanol and n-heptane of 3:7 (v/v).

### Examples

The abbreviations used include: dichloromethane (DCM), 4-N,N-dimethylaminopyridine (DMAP), hexamethyldisilazane (HMDS), ethanol (EtOH), ethyl acetate (AcOEt), methanol (MeOH), methyl (Me), ethyl (Et), isopropanol, phenyl (Ph), benzoyl (Bz), room temperature (rt or RT), triethylamine (TEA or Et₃N), tetrahydrofuran (THF).

### Example 1:

### Step a: Preparation (2R)-2-deoxy-2-fluoro-2-methyl-α/β-D-erythro-pentofuranosyl chloride-3,5-dibenzoate

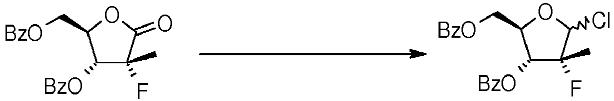

A solution of 132 g of trifluoroethanol in 110 g of toluene was slowly added at -30 to - 10°C to a solution of 381 g of Red-Al (Vitride®, 66.5% solution in toluene) in 90 g of toluene and the resulting mixture stirred for 30 minutes. The mixture was then allowed to warm to room temperature where it can be stored for several weeks.

114.7 g of this modified Red-Al reagent was added within 2 to 3 hours at -15 to -20°C to a suspension of 60 g of (2R)-2-deoxy-2-fluoro-2-methyl-D-ribonolactone-3,5-dibenzoate in 108 g of toluene and 78 g of butyl acetate and the resulting mixture was stirred for 1 to 2 hours. Upon reaction completion 0.6 g of tetrabutylammonium bromide was added. The solution was then treated at -20 to 0°C within 1 hour with 75.0 g of sulfuryl chloride. After addition completion, the mixture was warmed to 17 to 20°C and hold at this temperature for 4 to 5 h hours. The reaction mixture was then quenched by adding it at 15 to 40°C to a preformed solution of 180 g of sodium citrate dihydrate in 420 g of water. The first reactor and the transfer lines were rinsed with 60 g of butyl acetate. 38 g of sodium hydroxide (42% in water) was then added and the biphasic mixture was stirred for 1 hour at 30-35°C. The layers were allowed to settle for at least 30 minutes and the lower aqueous phase was removed. The organic layer was washed at 28-35°C with first an aqueous solution of 60 g of sodium citrate dihydrate in 140 g of water, followed by 200 g of water. From the organic layer water, toluene and butyl acetate were distilled off at a maximum temperature of 50°C and replaced by isopropyl acetate to afford 301.5 g of an isopropyl acetate solution containing 18.0 %(w/w) of the title compound as an α/(β-anomeric mixture (86% yield) which was used without further purification in the subsequent step b).

### Step b: Preparation of (2'R)-N-benzoyl-2'-deoxy-2'-fluoro-2'-methyl-cytidine-3',5'-dibenzoate

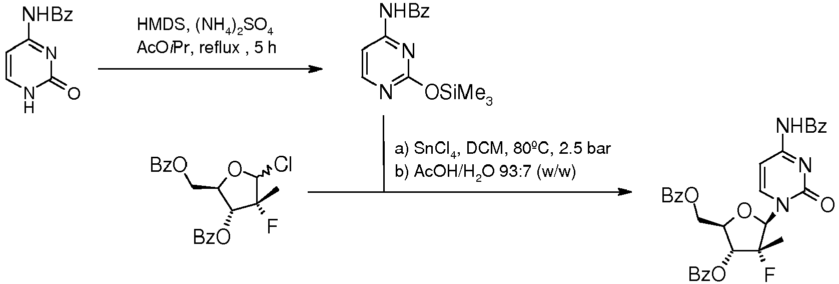

To a suspension of N-benzoyl cytosine (30.2 g, 140.3 mmol) and ammonium sulfate (400 mg, 2.8 mmol) in isopropyl acetate (320 mL) was added at reflux temperature within 30 to 60 minutes hexamethyldisilazane (22.5 g, 139.4 mmol) and the resulting mixture was stirred at reflux temperature (88-90°C) until a clear solution was obtained (approx. 5 hours). The solution was then concentrated under reduced pressure at approx. 40°C to a residual volume of approx. 90 mL. 200 g of (2R)- 2-deoxy-2-fluoro-2-methyl-α/β-D-erythro-pentofuranosyl chloride-3,5-dibenzoate (18 %(w/w) solution in isopropyl acetate; 91.3 mmol) was then added and the resulting mixture concentrated under reduced pressure at approx. 40°C to a residual volume of approx. 90 mL. The residue was treated with 200 mL of n-heptane and the solvents were completely removed at approx. 40°C to yield viscous oil. The oil was diluted with 340 mL of dichloromethane and the resulting turbid solution was treated at >30°C with tin tetrachloride (46.4 g, 178.1 mmol). The reactor was closed and the resulting mixture heated to 75-80°C (∼2.5 bars). The mixture was stirred at this temperature for 20 hours and subsequently cooled to room temperature. The reaction mixture was then added within 1 to 2 hours at 18 to 25°C to a preformed mixture of 72 g of acetic acid and 5.4 g water and the obtained grey suspension was subsequently stirred at 22°C for an additional hour. The suspension was filtered and the transfer lines and the filter cake were washed in portions with 160 mL of dichloromethane. To the filtrate water (170 mL) and acetic acid (170 mL) were added and the biphasic mixture was stirred for 20 minutes at 30°C. The layers were then allowed to separate for 30 minutes. The lower organic phase was separated and subsequently washed three times with a mixture of water (150 mL) and acetic acid (50 mL). The organic layer was then polish filtered (using a ZetaCarbonTM filter cartridge). The filtrate was diluted with 300 mL of methanol and from the resulting mixture dichloromethane/methanol was distilled off at atmospheric pressure and the removed solvent was continuously replaced by methanol keeping the volume in the reactor constant at 850-900 mL. The distillation was stopped when the batch temperature was 52°C. The resulting suspension was cooled to 20°C within 3 hours and stirred at this temperature for 2 hours. The crystals were filtered off, washed with methanol (200 mL) and dried at 55°C/<10 mbar to afford 34.9 g (67% yield) of the title compound with an assay (HPLC) of 99.8 %(w/w) and a tin contend of 9 ppm.

### Step c: Preparation of (2'R)-2'-deoxy-2'-fluoro-2'-methylcytidine

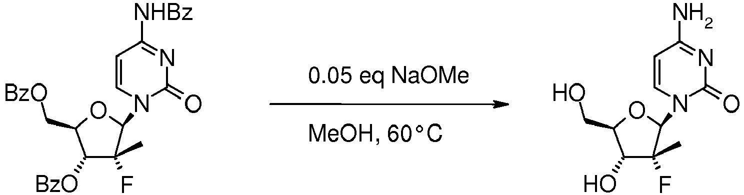

To a suspension of (2'R)-N-benzoyl-2'-deoxy-2'-fluoro-2'-methyl-cytidine-3',5'-dibenzoate (62.1 g, 108.6 mmol) in methanol (420 mL) was added at 60°C sodium methoxide (25 %(w/w) in methanol, 1.17 g, 5.4 mmol, 0.05 eq) and the resulting suspension was subsequently stirred at 60°C for 4 hours. Isobutyric acid (0.58 g, 6.5 mmol, 0.06 eq) was then added and the resulting mixture was polish filtered. From the filtrate methanol was distilled off at atmospheric pressure and the removed solvent was continuously replaced by isopropanol keeping the volume in the reactor constant at ∼300 mL. In total, 400 mL of isopropanol have been used for the solvent exchange. The resulting suspension was cooled from 80 to -2°C within 5 hours and subsequently stirred at this temperature for 4 hours. The crystals were filtered off, washed with isopropanol and dried at 70°C/<10 mbar to afford 25.6 g (91% yield) of the title compound with an assay (HPLC) of 99.6 %(w/w).

### Step d: Preparation of (2'R)-2'-deoxy-2'-fluoro-2'-methylcytidine-3',5'-diisobutyrate (Mericitabine)

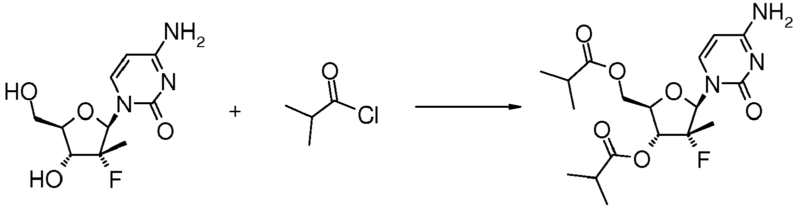

### Example 1:

In a jacketed vessel 20.0 g of (2'R)-2'-deoxy-2'-fluoro-2'-methylcytidine, 1.08 g of DMAP, and 54.4 g of TEA were suspended in 82.4 g of water and 184 g of THF and the mixture was cooled to 0 ± 5°C. 40 g of isobutyryl chloride were added within 1 to 2 hours at 0 ± 5°C. Upon complete addition, the solution was warmed to room temperature and the pH was adjusted to pH 6.0 to 7.0 with conc. hydrochloric acid. 120 g of ethyl acetate were then added and the biphasic mixture stirred for 20 minutes. The layers were allowed to separate for 20 minutes. The aqueous layer was separated (and discarded) and the organic layer was washed first with a mixture of 56 g of saturated aqueous sodium bicarbonate solution and 38 g of water followed by 72 g of water. The organic layer was concentrated to a volume of <50 ml under vacuum with a jacket temperature 50 to 70°C. 325 g of isopropanol were charged in portions while the solution was concentrated under vacuum. A total of 250 g of isopropanol was distilled from the vessel. The mixture was heated to 70-75°C and 275 g of n-heptane were added at this temperature within 3 to 4 hours. The formed suspension was then cooled to -5 to 0°C within 6 hours. After 2 hours stirring at this temperature, the crystals were filtered off, washed with a mixture of 10 g isopropanol and 30 g of n-heptane and dried at 50±5°C /<10 mbar to afford 26.7 g (86% yield) of the title compound with an assay (HPLC) of 99.3 %(w/w).

### Example 2:

In a 1000 mL double jacket reactor 40.0 g of (2'R)-2'-deoxy-2'-fluoro-2'-methylcytidine, 0.12 g of DMAP, and 98 g of TEA were added at 40°C to a mixture of 160 g of water and 350 g of THF. The resulting solution was cooled to 0 ±5°C and 68 g of isobutyryl chloride were added within 4 to 5 hours at 0±5°C. Upon complete addition, the solution was stirred for one additional hour at 0°C. The pH was then adjusted at 0°C with 20% aqueous sulfuric acid to pH 6.0 to 7.0. 150 g of ethyl acetate were added and the biphasic mixture stirred for 20 minutes at 0°C. The layers were allowed to separate for 20 minutes. The aqueous layer was separated (and discarded) and the organic layer was treated with 100 g of water and the pH of the mixture was adjusted at 0°C with 28% aqueous sodium hydroxide to pH 10.5 to 11.0. Ethyl acetate (110 g) was added and the biphasic mixture was allowed to warm to rt and stirred at this temperature for 2 hours. The layers were allowed to separate for 20 minutes. The aqueous layer was separated (and discarded). The organic layer was washed once with diluted aqueous sulfuric acid (110 g) and then with water (50 g). From the organic layer, ethyl acetate, THF, and water were completely removed by distillation and replaced by isopropanol. The resulting mixture (containing approx. 17 %(w/w) of the title compound) was heated to 65-70°C and 130 g of n-heptane were added at this temperature within 30 minutes. After seeding, the mixture was cooled to 55°C within 3 to 5 hours and 170 g of n-heptane were added at this temperature within one hour. The resulting suspension was then cooled to 0°C within 3 to 5 hours. At this temperature additional 600 g of n-heptane were added within one hour and the suspension stirred for 2 hours. The crystals were filtered off, washed with 150 g of n-heptane and dried at 50±5°C /<10 mbar to afford 55.2 g (90% yield) of the title compound with an assay (HPLC) of 99.4 % (w/w).

## Claims

1. A process for the preparation of the (2'R)-2'-deoxy-2'-fluoro-2'-methylcytidine derivative of formula I 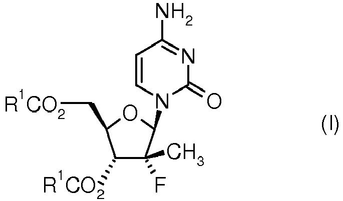 wherein R¹ is selected from C₁₋₄-alkyl,
comprising the steps
a) transforming the (2R)-2-deoxy-2-fluoro-2-methyl-D-ribonolactone derivative of formula II 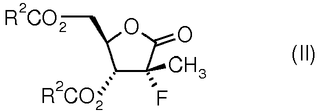 wherein R² is phenyl or C₁₋₄-alkyl
into the (2R)-2-deoxy-2-fluoro-2-methyl-D-ribofuranosyl chloride of formula III 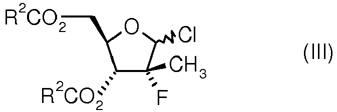 wherein R² is phenyl or C₁₋₄-alkyl
b) coupling the (2R)-2-deoxy-2-fluoro-2-methyl-D-ribofuranosyl chloride of formula III with N-benzoyl cytosine of formula VIa 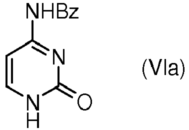 to form the (2'R)-N-benzoyl-2'-deoxy-2'-fluoro-2'-methyl-cytidine derivative of formula IV 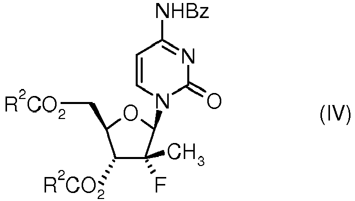 wherein R² is as above and Bz is benzoyl
c) alcoholysis of (2'R)-N-benzoyl-2'-deoxy-2'-fluoro-2'-methyl-cytidine derivative of formula IV to afford the (2'R)-2'-deoxy-2'-fluoro-2'-methyl-cytidine of formula V 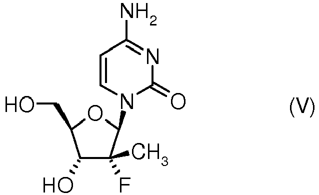 and
d) acylating the (2'R)-2'-deoxy-2'-fluoro-2'-methyl-cytisdine of formula V to form the (2'R)-2'-deoxy-2'-fluoro-2'-methylcytidine derivative of formula I,
**characterized in that** the coupling step b) comprises
b₁) the silylation of N-benzoyl cytosine of formula VIa 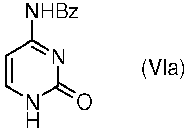 to form the silylated N-benzoyl cytosine of formula VIb 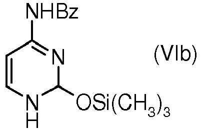 in the presence of a C₃₋₄-alkylacetate as solvent and
b₂) the coupling of the silylated N-benzoyl cytosine f formula VIb with the (2R)-2-deoxy-2-fluoro-2-methyl-D-ribofuranosyl chloride of formula III 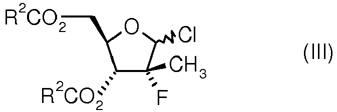 wherein R² is phenyl or C₁₋₄-alkyl,
to form the (2'R)-N-benzoyl-2'-deoxy-2'-fluoro-2'-methyl-cytidine derivative of formula IV 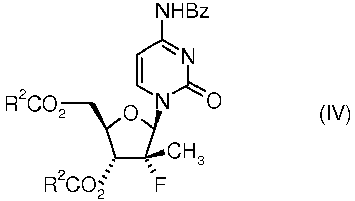 wherein R² is as above and Bz is benzoyl,
in the presence of dichloromethane as solvent and a Lewis acid.

2. Process according to claim 1, **characterized in that** R² is phenyl.

3. Process according to claim 1, **characterized in that** R¹ is i-propyl.

4. Process according to claim 1, **characterized in that** i-propyl- or n-butyl acetate is used as solvent for step b₁).

5. Process according to claims 1 and 4, **characterized in that** the silylation in step b₁) is performed with hexamethyldisilazane in the presence of ammonium sulfate.

6. Process according to claim 1, **characterized in that** the Lewis acid used in step b₂) is tin tetrachloride.

7. Process according to claims 1 and 6, **characterized in that** the coupling in step b₂) is performed at a reaction temperature of 70°C to 90°C and a pressure of 2 bar to 3 bar.

8. Process according to claim 1, 6 and 7, **characterized in that** the reaction mixture, after completion of the coupling reaction in step b₂) is quenched with a mixture of acetic acid and water of 97 : 3 (w/w) to 80 : 20 (w/w) at a temperature of 10°C to 30°C.

9. Process according to claim 1, 6, 7 and 8, **characterized in that** the (2'R)-N-benzoyl-2'-deoxy-2'-fluoro-2'-methyl-cytidine derivative of formula IV, obtained in step b₂) is further purified by multiple extractions of the tin with a mixture of water and acetic acid and subsequent crystallization by replacing partly of the dichloromethane by methanol.

10. Process according to claim 9, **characterized in that** the ratio of water and acetic acid for the extraction is 1 to 3 : 1 (v/v) and the ratio of methanol and dichloromethane for the crystallization is 2 to 5 : 1 (w/w).

11. Process according to claim 1, **characterized in that** the transformation in step a) comprises a reduction in the presence of a reducing agent and a subsequent chlorination in the presence of chlorinating agent.

12. Process according to claim 11, **characterized in that** the reducing agent is preformed from sodium bis-(2-methoxyethoxy) aluminum hydride und trifluoroethanol.

13. Process according to claim 11, **characterized in that** the chlorinating agent is selected from sulfuryl chloride, thionyl chloride or phosphorus oxychloride.

14. Process according to claim 13, **characterized in that** the chlorinating agent is sulfuryl chloride in the presence of catalytic amounts of tetrabutylammonium bromide.

15. Process according to claim 1, **characterized in that** the alcoholysis in step c) is performed in the presence of a base and an alcohol as solvent.

16. Process according to claim 15, **characterized in that** the base is sodium methoxide and the organic solvent is methanol.

17. Process according to claim 16, **characterized in that** 0.03 - 0.10 eq sodium methoxide is used at a reaction temperature of 50°C to 65°C.

18. Process according to claim 1, **characterized in that** the acylation in step d) is performed with a C₁₋₄-alkanoyl chloride in the presence of an organic solvent/water mixture at temperatures of -5°C and 5°C.

19. Process according to claim 18, **characterized in that** the C₁₋₄-alkanoylchloride is isobutyryl chloride and the organic solvent is tetrahydrofuran.

20. Process according to claim 18, **characterized in that** the (2'R)-2'-deoxy-2'-fluoro-2'-methylcytidine derivative of formula I obtained from step d) is crystallized in a mixture of a C₁₋₄-alcohol and n-heptane.

## Patentansprüche

1. Ein Verfahren zur Herstellung des (2'R)-2'-Desoxy-2'-fluor-2'-methylcytidin-Derivats der Formel I 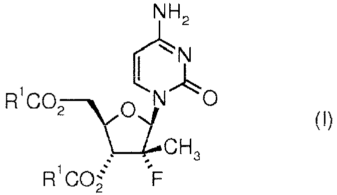 wobei R¹ ausgewählt ist aus C₁₋₄-Alkyl,
umfassend die Schritte
a) Umwandeln des (2R)-2-Desoxy-2-fluor-2-methyl-D-ribonolacton-Derivats der Formel II 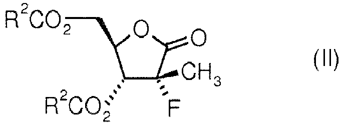 wobei R² Phenyl oder C₁₋₄-Alkyl ist,
in das (2R)-2-Desoxy-2-fluor-2-methyl-D-ribofuranosylchlorid der Formel III 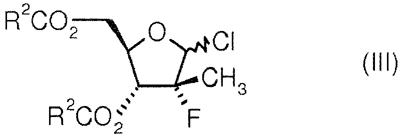 wobei R² Phenyl oder C₁₋₄-Alkyl ist,
b) Kuppeln des (2R)-2-Desoxy-2-fluor-2-methyl-D-ribofuranosylchlorids der Formel III mit N-Benzoylcytosin der Formel VIa 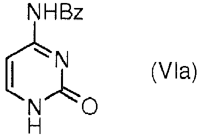 um das (2'R)-N-Benzoyl-2'-desoxy-2'-fluor-2'-methylcytidin-Derivat der Formel IV zu bilden, 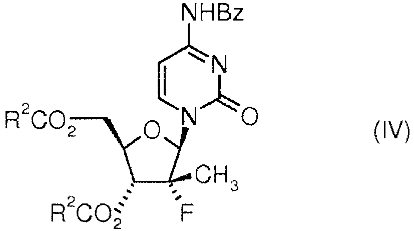 wobei R² wie vorstehend ist und Bz Benzoyl ist,
c) Alkoholyse des (2'R)-N-Benzoyl-2'-desoxy-2'-fluor-2'-methylcytidin-Derivats der Formel IV, um das (2'R)-2'-Desoxy-2'-fluor-2'-methyl-cytidin der Formel V bereitzustellen, 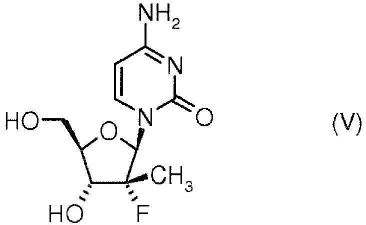 und
d) Acylieren des (2'R)-2'-Desoxy-2'-fluor-2'-methyl-cytidins der Formel V, um das (2'R)-2'-Desoxy-2'-fluor-2'-methylcytidin-Derivat der Formel I zu bilden,
**dadurch gekennzeichnet, dass** der Kupplungsschritt b)
b₁) das Silylieren von N-Benzoylcytosin der Formel VIa, 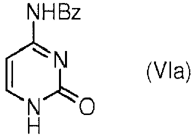 um das silylierte N-Benzoylcytosin der Formel VIb zu bilden, 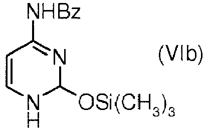 in Gegenwart eines C₃₋₄-Alkylacetats als Lösungsmittel und
b₂) das Kuppeln des silylierten N-Benzoylcytosins der Formel VIb mit dem (2R)-2-Desoxy-2-fluor-2-methyl-D-ribofuranosylchlorid der Formel III, 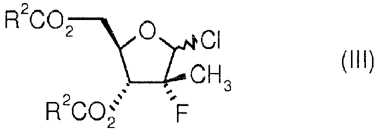 wobei R² Phenyl oder C₁₋₄-Alkyl ist,
um das (2'R)-N-Benzoyl-2'-desoxy-2'-fluor-2'-methylcytidin-Derivat der Formel IV zu bilden, 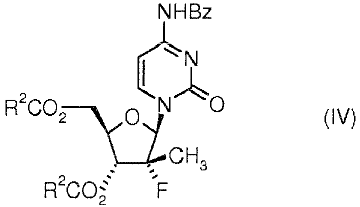 wobei R² wie vorstehend ist und Bz Benzoyl ist,
in Gegenwart von Dichlormethan als Lösungsmittel und einer Lewis-Säure umfasst.

2. Verfahren nach Anspruch 1, **dadurch gekennzeichnet, dass** R² Phenyl ist.

3. Verfahren nach Anspruch 1, **dadurch gekennzeichnet, dass** R¹ i-Propyl ist.

4. Verfahren nach Anspruch 1, **dadurch gekennzeichnet, dass** i-Propyl- oder n-Butylacetat als Lösungsmittel für Schritt b₁) verwendet wird.

5. Verfahren nach den Ansprüchen 1 und 4, **dadurch gekennzeichnet, dass** das Silylieren in Schritt b₁) mit Hexamethyldisilazan in Gegenwart von Ammoniumsulfat erfolgt.

6. Verfahren nach Anspruch 1, **dadurch gekennzeichnet, dass** die in Schritt b₂) verwendete Lewis-Säure Zinntetrachlorid ist.

7. Verfahren nach den Ansprüchen 1 und 6, **dadurch gekennzeichnet, dass** das Kuppeln in Schritt b₂) bei einer Reaktionstemperatur von 70°C bis 90°C und einem Druck von 2 bar bis 3 bar erfolgt.

8. Verfahren nach Anspruch 1, 6 und 7, **dadurch gekennzeichnet, dass** das Reaktionsgemisch nach Beendigung der Kupplungsreaktion in Schritt b₂) mit einem 97:3 (Gew./Gew.) bis 80:20 (Gew./Gew.)-Gemisch von Essigsäure und Wasser bei einer Temperatur von 10°C bis 30°C abgeschreckt wird.

9. Verfahren nach Anspruch 1, 6, 7 und 8, **dadurch gekennzeichnet, dass** das in Schritt b₂) erhaltene (2'R)-N-Benzoyl-2'-desoxy-2'-fluor-2'-methylcytiditi-Derivat der Formel IV weiter durch mehrfache Extraktionen des Zinns mit einem Gemisch von Wasser und Essigsäure und anschließende Kristallisation durch teilweises Ersetzen des Dichlormethans durch Methanol gereinigt wird.

10. Verfahren nach Anspruch 9, **dadurch gekennzeichnet, dass** das Verhältnis von Wasser und Essigsäure für die Extraktion 1 bis 3:1 (Vol./Vol.) beträgt und das Verhältnis von Methanol und Dichlormethan für die Kristallisation 2 bis 5:1 (Gew./Gew.) beträgt.

11. Verfahren nach Anspruch 1, **dadurch gekennzeichnet, dass** das Umwandeln in Schritt a) eine Reduktion in Gegenwart eines Reduktionsmittels und eine anschließende Chlorierung in Gegenwart eines Chlorierungsmittels umfasst.

12. Verfahren nach Anspruch 11, **dadurch gekennzeichnet, dass** das Reduktionsmittel aus Natriumbis-(2-methoxyethoxy)aluminiumhydrid und Trifluorethanol vorgebildet wird.

13. Verfahren nach Anspruch 11, **dadurch gekennzeichnet, dass** das Chlorierungsmittel aus Sulfurylchlorid, Thionylchlorid oder Phosphoroxychlorid ausgewählt ist.

14. Verfahren nach Anspruch 13, **dadurch gekennzeichnet, dass** das Chlorierungsmittel Sulfurylchlorid in Gegenwart katalytischer Mengen an Tetrabutylammoniumbromid ist.

15. Verfahren nach Anspruch 1, **dadurch gekennzeichnet, dass** die Alkoholyse in Schritt c) in Gegenwart einer Base und eines Alkohols als Lösungsmittel erfolgt.

16. Verfahren nach Anspruch 15, **dadurch gekennzeichnet, dass** die Base Natriummethoxid ist und das organische Lösungsmittel Methanol ist.

17. Verfahren nach Anspruch 16, **dadurch gekennzeichnet, dass** 0,03-0,10 Äq. Natriummethoxid bei einer Reaktionstemperatur von 50°C bis 65°C verwendet werden.

18. Verfahren nach Anspruch 1, **dadurch gekennzeichnet, dass** das Acylieren in Schritt d) mit einem C₁₋₄-Alkanoylchlorid in Gegenwart eines Gemischs von organischem Lösungsmittel/Wasser bei Temperaturen von -5°C und 5°C erfolgt.

19. Verfahren nach Anspruch 18, **dadurch gekennzeichnet, dass** das C₁₋₄-Alkanoylchlorid Isobutyrylchlorid ist und das organische Lösungsmittel Tetrahydrofuran ist.

20. Verfahren nach Anspruch 18, **dadurch gekennzeichnet, dass** das aus Schritt d) erhaltene (2'R)-2'-Desoxy-2'-fluor-2'-methylcytidin-Derivat der Formel I in einem Gemisch von einem C₁₋₄-Alkohol und n-Heptan kristallisiert wird.

## Revendications

1. Procédé de préparation du dérivé de (2'R)-2'-désoxy-2'-fluoro-2'-méthylcytidine de formule I 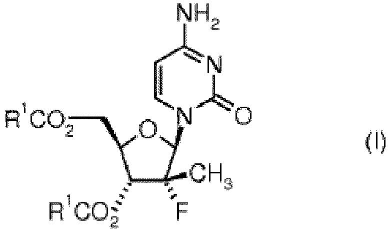 dans laquelle R¹ est choisi parmi un alkyle en C₁ à C₄,
comprenant les étapes suivantes
a) la transformation du dérivé de (2R)-2-désoxy-2-fluoro-2-méthyl-D-ribonolactone de formule II 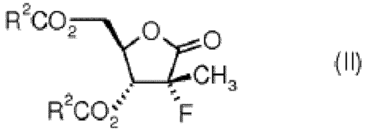 dans laquelle R² est un phényle ou un alkyle en C₁ à C₄
en chlorure de (2R)-2-désoxy-2-fluoro-2-méthyl-D-ribofuranosyle de formule III 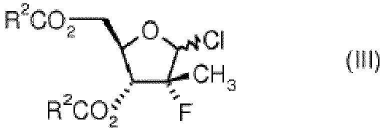 dans laquelle R² est un phényle ou un alkyle en C₁ à C₄
b) le couplage du chlorure de (2R)-2-désoxy-2-fluoro-2-méthyl-D-ribofuranosyle de formule III avec la N-benzoylcytosine de formule VIa 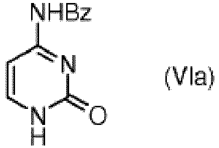 pour former le dérivé de (2'R)-N-benzoyl-2'-désoxy-2'-fluoro-2'-méthyl-cytidine de formule IV 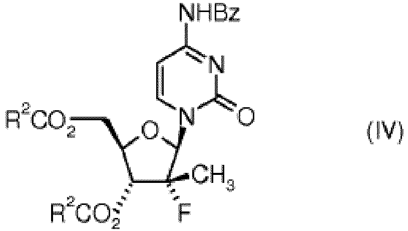 dans laquelle R² est tel que ci-dessus et Bz est un benzoyle
c) l'alcoolyse du dérivé de (2'R)-N-benzoyl-2'-désoxy-2'-fluoro-2'-méthyl-cytidine de formule IV pour obtenir la (2'R)-2'-désoxy-2'-fluoro-2'-méthyl-cytidine de formule V 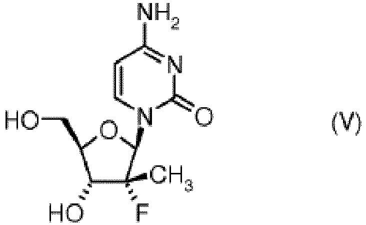 et
d) l'acylation de la (2'R)-2'-désoxy-2'-fluoro-2'-méthyl-cytidine de formule V pour former le dérivé de (2'R)-2'-désoxy-2'-fluoro-2'-méthylcytidine de formule I,
**caractérisé en ce que** l'étape b) de couplage comprend
b₁) la silylation de la N-benzoylcytosine de formule VIa 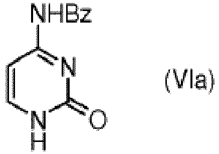 pour former la N-benzoylcytosine silylée de formule VIb 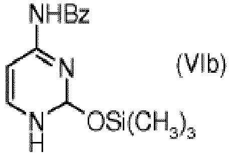
en présence d'un acétate d'alkyle en C₃ à C₄ jouant le rôle de solvant, et
b₂) le couplage de la N-benzoylcytosine silylée de formule VIb avec le chlorure de (2R)-2-désoxy-2-fluoro-2-méthyl-D-ribofuranosyle de formule III 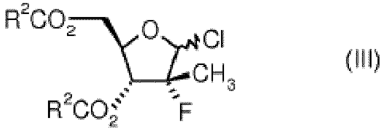 dans laquelle R² est un phényle ou un alkyle en C₁ à C₄,
pour former le dérivé de (2'R)-N-benzoyl-2'-désoxy-2'-fluoro-2'-méthyl-cytidine de formule IV 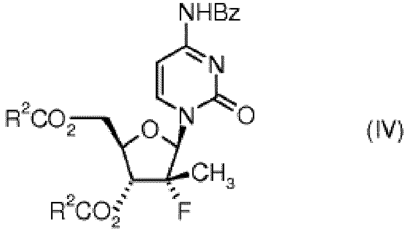 dans laquelle R² est tel que ci-dessus et Bz est un benzoyle,
en présence de dichlorométhane jouant le rôle de solvant et d'un acide de Lewis.

2. Procédé selon la revendication 1, **caractérisé en ce que** R² est un phényle.

3. Procédé selon la revendication 1, **caractérisé en ce que** R¹ est un i-propyle.

4. Procédé selon la revendication 1, **caractérisé en ce qu'**un acétate d'i-propyle ou de n-butyle est utilisé comme solvant pour l'étape b₁).

5. Procédé selon les revendications 1 et 4, **caractérisé en ce que** la silylation dans l'étape b₁) est réalisée avec de l'hexaméthyldisilazane en présence de sulfate d'ammonium.

6. Procédé selon la revendication 1, **caractérisé en ce que** l'acide de Lewis utilisé dans l'étape b₂) est le tétrachlorure d'étain.

7. Procédé selon les revendications 1 et 6, **caractérisé en ce que** le couplage dans l'étape b₂) est réalisé à une température réactionnelle de 70 °C à 90 °C et sous une pression de 2 bar à 3 bar.

8. Procédé selon les revendications 1, 6 et 7, **caractérisé en ce que** le mélange réactionnel, après la fin de la réaction de couplage dans l'étape b₂), est désactivé avec un mélange d'acide acétique et d'eau de 97/3 (en poids/poids) à 80/20 (en poids/poids) à une température de 10 °C à 30 °C.

9. Procédé selon les revendications 1, 6, 7 et 8, **caractérisé en ce que** le dérivé de (2'R)-N-benzoyl-2'-désoxy-2'-fluoro-2'-méthyl-cytidine de formule IV, obtenu dans l'étape b₂), est en outre purifié par de multiples extractions de l'étain avec un mélange d'eau et d'acide acétique, puis par cristallisation en remplaçant partiellement le dichlorométhane par du méthanol.

10. Procédé selon la revendication 9, **caractérisé en ce que** le rapport eau sur acide acétique pour l'extraction est de 1 à 3/1 (en volume/volume) et que le rapport méthanol sur dichlorométhane pour la cristallisation est de 2 à 5/1 (en poids/poids).

11. Procédé selon la revendication 1, **caractérisé en ce que** la transformation dans l'étape a) comprend une réduction en présence d'un agent réducteur, puis une chloration en présence d'un agent de chloration.

12. Procédé selon la revendication 11, **caractérisé en ce que** l'agent réducteur est préalablement formé à partir de l'hydrure de bis-(2-méthoxyéthoxy)aluminium et de sodium et du trifluoroéthanol.

13. Procédé selon la revendication 11, **caractérisé en ce que** l'agent de chloration est choisi parmi le chlorure de sulfuryle, le chlorure de thionyle ou l'oxychlorure de phosphore.

14. Procédé selon la revendication 13, **caractérisé en ce que** l'agent de chloration est le chlorure de sulfuryle en présence d'une quantité catalytique de bromure de tétrabutylammonium.

15. Procédé selon la revendication 1, **caractérisé en ce que** l'alcoolyse dans l'étape c) est réalisée en présence d'une base et d'un alcool jouant le rôle de solvant.

16. Procédé selon la revendication 15, **caractérisé en ce que** la base est le méthoxyde de sodium et le solvant organique est le méthanol.

17. Procédé selon la revendication 16, **caractérisé en ce que** 0,03 éq à 0,10 éq de méthoxyde de sodium est utilisé à une température réactionnelle de 50 °C à 65 °C.

18. Procédé selon la revendication 1, **caractérisé en ce que** l'acylation dans l'étape d) est réalisée avec un chlorure d'alcanoyle en C₁ à C₄ en présence d'un mélange solvant organique/eau à des températures de -5 °C et 5 °C.

19. Procédé selon la revendication 18, **caractérisé en ce que** le chlorure d'alcanoyle en C₁ à C₄ est le chlorure d'isobutyryle et le solvant organique est le tétrahydrofuranne.

20. Procédé selon la revendication 18, **caractérisé en ce que** le dérivé de (2'R)-2'-désoxy-2'-fluoro-2'-méthylcytidine de formule I obtenu dans l'étape d) est cristallisé dans un mélange contenant un alcool en C₁ à C₄ et du n-heptane.
